# EUROPEAN PATENT APPLICATION

(11) **EP 4 248 842 A1**
(43) Date of publication of application: **27.09.2023**
(21) Application number: 22164342.2
(22) Date of filing: 25.03.2022
(51) Int. Cl.: A61B 5/00

(54) **PERSONALIZED PATIENT HYDRATION STATE MEASURING DEVICE**

(71) Applicant: Fresenius Medical Care Deutschland GmbH, 61352 Bad Homburg (DE)
(72) Inventor: Gervasoni, Federica, 61352 Bad Homburg (DE); Neri, Luca, 61352 Bad Homburg (DE); Bellocchio, Francesco, 61352 Bad Homburg (DE); Kuss, Matthias, 61352 Bad Homburg (DE)
(74) Representative: Bobbert & Partner Patentanwälte PartmbB

(57) **Abstract**

The present invention relates to a method for generating at least one data set to be used for a hydration measurement device (200) when measuring a hydration state of the patient. The method encompasses the step of providing a data set generating device (510), comprising at least one algorithm, or data, for generating a data set based on a measured hydration value of the patient on a first body part and a determined reference hydration value from a reference hydration data source (250) selected with regard to collected further patient-specific data, the data set being usable for determining a reliable hydration value of a second body part of the patient or the overall hydration value when measuring with the hydration measurement device (200) on the first body part of the patient. Storing the data set in a data set storage (220) is also encompassed by the method.

## Description

The present invention relates to a method for generating data sets according to claim 1, a hydration measurement device according to claim 9 and a set according to claim 14. Furthermore, it relates to a computer program product or a computer program according to claim 15. Alternatively the present invention may relate to the preambles or generic terms of each of these claims.

From the prior art hydration measurement devices are known for determining the hydration status of a patient. The technical basis for determining the former is disclosed in, e.g., WO 2006/002685 A1. Such measurement devices have to be configured or programmed in order to achieve high quality measurements.

An object of the present invention may be to suggest a method for hydration measurement devices for determining a hydration status of a patient, a hydration measurement device, and a set comprising the measurement device and at least one more item.

Moreover, a computer program product and a computer program should be suggested.

The objective of the present invention may be solved by the method for generating data sets having the features of claim 1. It may further be achieved by the hydration measurement device having the features of claim 9 and the set having the features of claim 14. Furthermore, it may be achieved by the computer program product or by the computer product having the features of claim 15.

According to the present invention, a method, in particular a computer-implemented method, is suggested. The method serves for generating data sets for a, in particular specific, hydration measurement device to be used when measuring a hydration state of a patient.

The method according to the present invention encompasses a step of providing or using a hydration measurement device. The hydration measurement device is suitable and/or configured for measuring at least one hydration parameter of a first body part or body region of the patient. Optionally, the hydration measurement device is a wearable, optionally, it is comprised by or integrated in a wearable.

As a further step the method encompasses a measuring of the at least one hydration parameter of the patient using the hydration measurement device. This results in obtaining a measured hydration value of or on that first body part or body region of the patient. Alternatively, such a measured hydration value, e.g. measured prior to the method by the hydration measurement device, is provided.

Whenever the term "hydration value" is mentioned herein it encompasses at least overhydration as well as underhydration. The hydration status may be considered a hydration status of the patient's blood and/or hydration status of the patient's body tissue.

A data set generating device comprising at least one algorithm, or data, for generating a data set, or the use of such a device, is provided by the method.

As a further step, collecting patient-specific data of the (specific, i.e., particular) patient is encompassed by the method.

Whenever "patient-specific data" is mentioned herein this may include any parameter, data or value that may be determined, measured, collected, captured etc. on or with the patient, e.g. his/her blood pressure, body temperature, pulse, weight, height, sex, age and so on.

Determining, selecting or retrieving at least one reference hydration value from a reference hydration data source based on the patient-specific data of the patient is a further step of the method.

The method further includes the step of reading-in the at least one hydration value of the patient measured by the hydration measurement device and/or the at least one reference hydration value determined from the reference hydration data source into the data set generating device. This is preferably carried out using a reading device.

Based on the read-in measured hydration value and/or the at least one determined reference hydration value, a data set specific for the patient is generated by the data set generating device. Herein, the data set is usable for determining or estimating a hydration value of the patient on or of at least one second body part or body region of the patient or for determining the overall hydration value of the patient when using the hydration measurement device in the future.

Finally, storing the generated data set in a data set storage is also encompassed by the method according to the present invention.

According to the present invention, a hydration measurement device for measuring at least one hydration parameter of a first body part of the patient is suggested.

The hydration measurement device is optionally a wearable, optionally, it is comprised by or integrated in a wearable, e.g. a wrist watch, a wrist band smart watch, a fitness tracker or the like.

Furthermore, the hydration measurement device is configured for attributing or determining a hydration value of or on a second body part or body region of the patient or the overall hydration of the patient to the hydration value measured by means of the hydration measurement device using at least one data set generated by the method according to the present invention.

According to the present invention, further a set is suggested, the set comprising a hydration measurement device, preferably according to the present invention, and also at least one further item from the following group of items:
- a reference hydration measurement device;
- a computing device comprising software for executing the computational steps of the method according to the present invention; and/or
- a data set storage comprising, or configured to comprise, at least one data set generated based on read-in measured hydration values, reference hydration values, patient-specific data and/or device-specific data.

According to the present invention, a computer program-product having a program code stored on a machine readable carrier configured for carrying out or initiating the computational steps of the method according to the present invention is suggested.

When the term "computational steps (of the method)" is mentioned herein, it comprises all method steps that can be run on or executed by a computer program on a computer, in particular that can be executed without human intervention.

A computer program product according to the present invention, comprises a volatile or transient program code or one stored on a machine-readable program carrier or a signal wave, by which the method steps of the method according to the present invention, in particular the computational steps thereof, are initiated or carried out when the computer program product is running on a computer. A computer program product may be understood according to the present invention as e.g. a computer program stored on a carrier, an embedded system being a comprehensive system with a computer program (e.g., an electronic device with a computer program), a network of computer implemented computer programs (e.g. client/server-system, a cloud computing system etc.), or a computer on which a computer program is loaded, runs, is stored, is being executed or developed.

The term "machine readable program carrier" as it is used herein, refers in certain embodiments of the present invention to a carrier, which contains data or information interpretable by software and/or hardware. The carrier may be a data carrier, such as a diskette, a CD, DVD, a USB stick, a flashcard, an SD card or the like, as well as any other storage referred to herein or any other storage medium referred to herein.

Furthermore, according to the present invention, a computer program with a program code configured for carrying out or initiating the computational steps of the method according to the present invention is suggested.

A computer program according to the present invention encompasses a program code by which the method steps of the method according to the present invention, in particular the computational steps thereof, are initiated or carried out, when the computer program runs on a computer.

Embodiments according to the present invention may comprise one or several of the features mentioned supra and/or in the following in any combination unless the person skilled in the art considers the particular combination to be technically impossible.

Advantageous developments of the present invention are each subject-matter of the dependent claims and embodiments.

In all of the aforementioned and following statements, the use of the expression "may be" or "may have" and so on, is to be understood synonymously with "preferably is" or "preferably has", and so on respectively, and is intended to illustrate an embodiment according to the present invention.

Whenever alternatives with "and/or" are introduced herein, the person skilled in the art will understand the "or" contained therein as preferably "either or" and preferably not as "and".

Whenever numerical words are mentioned herein, the person skilled in the art shall recognize or understand them as indications of numerical lower limits. Hence, unless this leads to a contradiction evident for the person skilled in the art, the person skilled in the art shall comprehend for example "one" (or "a/an") as encompassing "at least one". This understanding is also equally encompassed by the present invention as the interpretation that a numerical word, for example, "one" (or "a/an") may alternatively mean "exactly one", wherever this is evidently technically possible in the view of the person skilled in the art. Both of these understandings are encompassed by the present invention and apply herein to all used numerical words.

In case of doubt, the person skilled in the art will understand spatial information like "top", "bottom", "upper", "lower", "left" or "right" whenever they are herein mentioned, as a spatial indication with reference to the alignment in the figures appended hereto. "Bottom" is closer to the centre of the earth or to the bottom of the figure than "top".

Whenever "a reference hydration value" is mentioned herein, the term may encompass values, in particular equivalent values, functions and/or algorithms that may serve for enhancing the accuracy of a measured hydration value, measured by the hydration measurement device.

Whenever an embodiment is mentioned herein, it represents an exemplary embodiment according to the present invention.

When it is disclosed herein that the subject-matter according to the present invention comprises one or several features in a certain embodiment, it is also respectively disclosed herein that the subject-matter according to the present invention does, in other embodiments, likewise according to the present invention, explicitly not comprise this or these features, for example, in the sense of a disclaimer. Therefore, for every embodiment mentioned herein it applies that the converse embodiment, e.g. formulated as negation, is also disclosed.

When method steps are mentioned herein, then the set according to the present invention or at least one of its devices is in several embodiments configured in order to execute, in any combination, one or several of these method steps, in particular when these are automatically executable steps, or in order to accordingly control corresponding apparatuses which preferably correspond with their names to the designation of the respective method step (for example, "determining" as a method step and "apparatus for determining" for the apparatus) and which may likewise be part of the set according to the present invention or at least one of its devices or may be connected thereto in signal communication.

When programmed or configured is mentioned herein, then these terms may in some embodiments be interchangeable.

When a signal communication or communication connection between two components is mentioned herein, this may be understood to mean a connection that exits during use. It may also be understood that a preparation for such a (wired, wireless or otherwise implemented) signal communication exists, for example by coupling both components, for example by pairing, etc.

Pairing is a process that takes place in connection with computer networks in order to establish an initial link between computer units for the purpose of communication. The best-known example of this is the establishing of a Bluetooth connection, by which various devices (e.g. smartphone, headphones) are connected to each other. Pairing is sometimes also referred to as bonding.

The control device or closed-loop control device may prompt the execution of all or substantially all of the method steps or of the computational steps. The method according to the present invention may substantially or completely be executed by the control device or closed-loop control device. It may be partly executed by the control device or closed-loop control device. Such method steps may optionally be restricted to machine-induced or automatic method steps.

In some embodiments the reference hydration data source used with the method according to the present invention is a reference hydration measurement device, e.g. a body composition monitor (short: BCM) or the like. In these embodiments, the reference hydration value under consideration is at least one reference hydration value measured by the reference hydration measurement device on or of at least one second body part of the patient.

In several embodiments, the method encompasses the step of providing a measuring standard ("MESSNORMAL") or using such a device. This measuring standard, which can be part of the set according to the present invention, is being used for calibrating the hydration measurement device at least once, e.g. during manufacture, before delivery or before commissioning.

In some embodiments, the method encompasses a step of collecting device-specific data of the hydration measurement device. In these embodiments, the generation of the data set, using the data set generating device, is carried out specific also for the hydration measurement device by basing the generation also on the device-specific data. Device-specific data may comprise, e.g., the make, the model-type, any particularities or distinguishing features, the design (a body weight scale, a watch, or the like).

In several embodiments, the method encompasses the step of providing or using a retrieving device for retrieving the data set specific for the hydration measurement device, the data set being stored in the data set storage, from the data set storage and the step of reading in, into the retrieving device, at least one hydration value of the patient measured by the hydration measurement device.

In certain embodiments, prior to this step a further method step may be carried out, namely, measuring at least one value related to the hydration of the first body part or body region of the patient by the hydration measurement device.

Based on the retrieved data set specific for the hydration measurement device and the read-in at least one hydration value of the first body part or region of the patient, a hydration value of the patient relating to his/her at least one second body part of the patient, or the overall hydration value of the patient, is determined.

In some embodiments the such determined hydration value of the patient may be output to the patient or to another person, device, storage or the like, e.g. via the hydration measurement device or via another outputting device.

In several embodiments the determined hydration value of the patient may be output to a member of the medical staff.

In some embodiments the such determined hydration value of the patient may be stored in a storage device in order to be retrieved at a certain point in time, e.g., during a following treatment session. Providing devices suitable and/or configured for this purpose or control devices thereof are also encompassed by the present invention.

In several embodiments the determined hydration value of the patient is output instead of the measured hydration value, in others it is output in addition to it.

In some embodiments, the method according to the present invention encompasses the step of measuring another hydration value on or of the at least one second body part or body region of the patient or measuring the overall hydration value of the patient, e.g. by using a body composition monitor, while obtaining a measured reference hydration value of the patient.

In these embodiments of the method, the data set may be evaluated based on the deviation between the measured reference hydration value and the determined hydration value of the patient. It is then intended, if applicable, to generate an update of the data set by the data set generating device based on the result of the evaluation and, after generation, to store the updated data set in the data set storage, in particular replacing the former data set.

In several embodiments of the method, the hydration measurement device is configured to measure via optical methods, in particular via optical spectrometry.

In some embodiments of the method, the data set storage is comprised by the hydration measurement device.

In several embodiments, the data set storage is in signal communication with the retrieving device of the hydration measurement device.

In some embodiments, the data set storage is in signal communication with the reading device of the data set generating device.

In several embodiments of the method, the data set generating device is programmed to use data analyzing methods, in particular when generating an updated data set for the patient. Such analyzing methods may comprise sophisticated gold-standard analytic techniques, such as artificial intelligence (AI), neural networks, cutting-edge algorithms, mathematical functions and integral transformations.

In some embodiments of the method, the at least one algorithm, or data, is programmed to calculate a data set or an updated data set based on a multitude of values comprised by the patient-specific data, optionally weighted. Those values can comprise the blood pressure, body temperature, pulse, weight, height, sex, age and/or any patient-specific data.

In several embodiments the hydration measurement device, preferably according to the present invention, with which at least one hydration parameter of a first body part of the patient can be measured, comprises a reading device programmed for reading-in the data set from the data set storage comprised by the hydration measurement device.

In some embodiments, the hydration measurement device, preferably according to the present invention, with which at least one hydration parameter of a first body part of the patient is measured, comprises a reading device. The reading device is programmed for reading-in the data set from a data set storage not comprised by the hydration measurement device, e.g. the data set storage being implemented in a network, a cloud or the like.

In such embodiments, the data set storage may also be denoted as external storage device. This may allow storing data sets for a plurality of different hydration measurement devices of different, particular patients in one location. A corresponding data set may be accessible by the hydration measurement device or a retrieving device thereof to which it has been previously assigned.

In order to implement such an external storage, cloud computing would be advantageous due to the following reasons:
Cloud computing makes it possible to provide, on demand, - usually via the Internet and independent of devices - shared computer resources as services, for instance as servers, data storage or applications, promptly and with little effort. The offer and use of these computer resources is defined and usually takes place via an application programming interface (API) or via a website or app. The API is usually a set of commands, functions, protocols and objects that programmers may use to create software or interact with an external system, while the website or app, which may likewise be accessed from an external system, may provide applications for an (end) user to use them without further programming knowledge.

In this, services, which are available unilaterally and without human interaction, can be provided, on demand, in the cloud and can be called up for "self-service", so to speak, by an external system (keyword: on-demand self-service).

In addition, services may be accessed via a network with standard mechanisms, in particular using end devices such as smartphones, tablets, notebooks or workstations (keyword: broad network access).

Furthermore, computer resources may be pooled to serve multiple users as needed (keyword: resource pooling).

Services may be provided and released elastically in order to scale up and down as needed, in some cases automatically. From the user's point of view, the available computer resources may then appear unlimited and services may be modified or adapted in any way at any time (keyword: rapid elasticity).

Cloud computing also makes it possible to control and optimize computer resources on the basis of measurable figures that are collected depending on the respective performance, such as memory, bandwidth or active user accounts (keyword: measured service).

In edge computing, computer applications, data and services are moved away from central nodes, i.e. to the network periphery. For example, calculations are thereby performed decentrally, where the data actually originates or is collected and/or is required, with the aim of being able to work in a resource-saving manner and to prevent time delays caused by data transmission over a physical distance, i.e. to shorten response times.

In this, edge computing makes less demands on the bandwidth of the network connection used, together with cost savings due to lower data volumes and to the lower real-time demand on the network connection.

In both cloud computing and edge computing, a so-called remote procedure call may be implemented via a software, which makes it possible to call up system-remote functions, which are then executed on the remote software system. The result of such functions may then be transmitted back to the calling system.

In several embodiments, the hydration measurement device, preferably according to the present invention, is configured for executing the method, in particular as a computer-implemented method, according to the present invention or the computational steps thereof. Therewith, generating the data set based on at least a read-in measured hydration value, at least one reference hydration value, patient-specific data and device-specific data.

In some embodiments, the hydration measurement device is configured to measure via optical methods, in particular via optical spectrometry.

In several embodiments, the hydration measurement device comprises a computer program for executing the steps or the computational steps of the method according to the present invention.

In some embodiments, single, several or all devices for executing the method, in particular the data set generating device and/or the data set storage, may be implemented or located in a cloud or network. In these embodiments, the storage content, in particular a specific single data set, is advantageously retrievable by different (specific) hydration measurement devices and/or for different (specific) patients. The respective retrieved data set is provided and/or suitable to be processed in the (specific) hydration measurement device of the (particular) patient.

In several embodiments, outputs may be effected on different end devices, e.g. a smartphone and/or a tablet of the patient and/or of caregivers.

In some embodiments, a digital, particularly non-volatile storage medium, according to the present invention, particularly in the form of a machine readable carrier, particularly in the form of a diskette, CD, DVD, EPROM, FRAM (Ferroelectric RAM) or SSD (Solid State Drive), particularly with electronically or optically readable control signals, can interact with a programmable computer system, such that the method according to the present invention may be carried out or initiated.

The present invention, in some embodiments, does not include the method step related to the deductive medical or veterinary decision phase, i.e. the diagnosis stricto sensu representing a purely intellectual exercise.

In certain embodiments, the method does neither pertain to the diagnosis for curative purposes as a purely intellectual exercise representing the deductive medical or veterinary decision phase, nor encompass the features relating to the preceding steps which are constitutive for making the diagnosis.

Some or all of the embodiments according to the present invention may comprise one, several or all of the advantages mentioned above and/or in the following.

In certain embodiments, the hydration state, in particular the overall hydration value, which can display an overhydration of the patient, is approximated, calculated or defined based on measured values and/or calculations reflecting the overhydration (OH) or the relative overhydration (relOH: overhydration (OH) over extracellular water (ECW)), etc. of the patient. With regards to a definition of overhydration (OH), it is referred to WO 2006/002685 A1 where OH equals a*ECW + b*ICW + c*body weight. The respective disclosure of WO 2006/002685 A1 is hereby incorporated by way of reference. It is to be understood that the reference hydration value can be determined in different ways, all of which are known to the person skilled in the art. One of those methods comprises measuring of a dilution and calculating, e.g., an overhydration based thereon.

An advantage of the present invention may be that hydration measurement devices, for example, intended for use in the LifeScience or leisure sector, which may be used e.g. for interdialytical measurements, are configured in a way that determined hydration values are sufficiently accurate to be used for medical purposes. An exemplary purpose may be the determination of a therapeutic ultrafiltration volume, e.g. in an upcoming dialysis treatment session. This may help to enhance the patient safety and the patient compliance.

As these LifeScience and leisure measurement devices offer the possibility of a comfortable long term patient monitoring (e.g. by wristbands, smartwatches, chest straps and/or other wearables) at low costs, the use of the present invention may advantageously help to save costs.

Another advantage of the present invention may be that optical measurement devices neither influence a current dialysis treatment session nor may be influenced by the latter. A measurement during dialysis, although preferably not comprised by the present invention, is, therefore, easily possible, rather than it is the case when using e.g. bioimpedance measurement devices which measure electrically.

By using the present invention, also statements about the total hydration state of a patient may advantageously be made, although measurement devices are used that only measure selectively on specific body sections, e.g. the patient's wrist. Therefore, as an example, by a measurement at the patient's wrist, the hydration state (hyperhydration/ hypohydration) of the patient may be determined, alternatively, the hydration state of other body segments. Thereby, all possible measured or determined parameters of the patient, such as vital parameters or other patient-specific parameters, may be taken into account, e.g. blood pressure, body temperature, pulse, weight, sex, height, age, etc.

Another advantage of the present invention may be, that its functions and/or algorithms may be parameterizable, i.e. they may be adjusted to a specific measurement device. This may advantageously allow to compensate for manufacturing tolerances between devices of the same type.

Advantageously, the present invention may result in technical enhanced new measurement devices. This may in particular be the case if the data sets are stored and/or processed in the hydration measurement device itself and lead to a more general assessment.

All the advantages achievable with the method according to the present invention can be in certain embodiments achieved undiminished by the apparatuses or devices according to the present invention, and vice versa.

In the following, the present invention is described based on preferred embodiments thereof with reference to accompanying drawing. However, the present invention is not to be limited to these embodiments. In the figures, in which same reference numerals denote identical or similar elements, values and so on, the following applies:
- **Fig. 1**: shows schematically simplified a course of the method according to the present invention in an exemplary embodiment; and
- **Fig. 2**: shows schematically simplified a set according to the present invention in an exemplary embodiment.

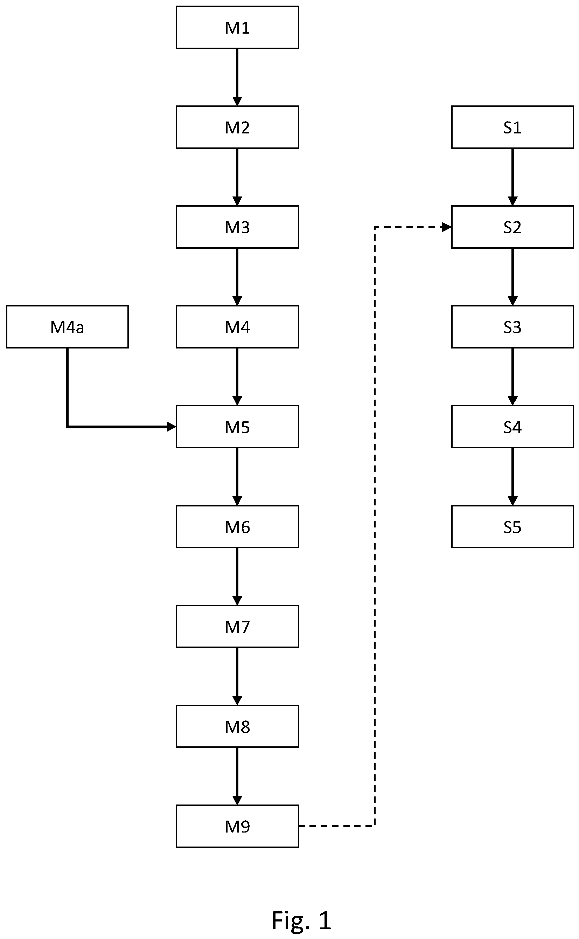

**Fig. 1** shows schematically simplified a course of the method according to the present invention in an exemplary embodiment.

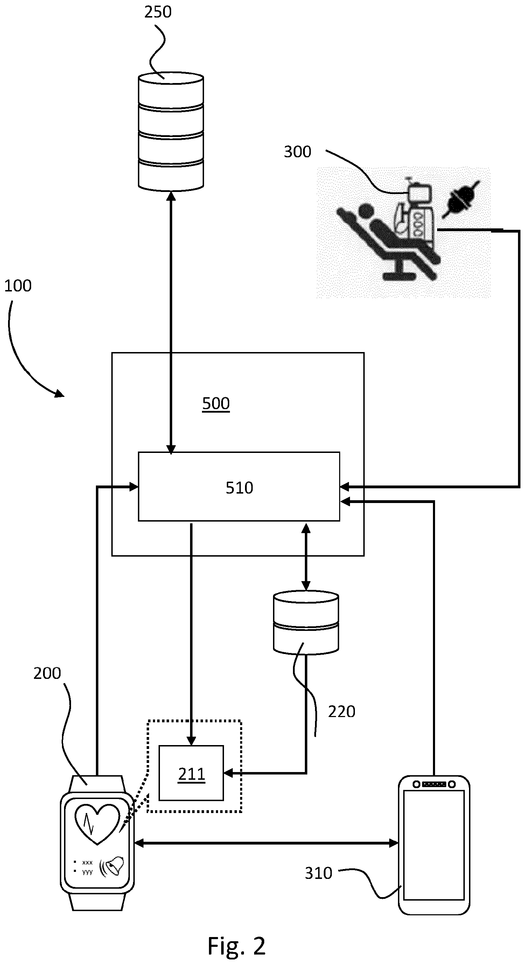

Reference is made to the reference numerals of the devices which are described in more detail with regard to Fig. 2.

The method, which may in particular be computer-implemented, aims at generating data sets for a hydration measurement device 200. The generated data set may be used when measuring and/or monitoring a hydration state of a patient using the hydration measurement device 200. The method encompasses as a first, optional method step M1, providing a hydration measurement device 200 for measuring and/or monitoring at least one hydration parameter of a first body part or body region of a patient, and measuring the at least one hydration parameter of the first body part or body region of the patient while obtaining a measured hydration value of the first body part or body region in a second, optional method step M2. Alternatively, instead of providing the hydration measurement device 200 and measuring said hydration value, a hydration value already measured using the hydration measurement device 200 is provided, and only its use is encompassed by the method. Hence, the measured hydration value can already have been at hand before even starting the method.

In the example of Fig. 2, the hydration measurement device 200 is a wearable or comprised by a wearable, here a patient's smart watch.

Providing a data set generating device 510 comprising at least one algorithm, or data, for generating a data set is further encompassed as method step M3 by the method. Alternatively, the method encompasses the use of such a device.

Method step M4 represents the collection of patient-specific data of the patient, in particular in the form as specified herein (weight, sex, age, temperature, etc.).

In some embodiments of the method according to the present invention, additionally, device-specific data of the hydration measurement device 200 is collected. This is represented in Fig. 1 by the method step M4a.

As method step M5 determining or selecting or retrieving at least one reference hydration value from a reference data source based on the patient-specific data of the patient is encompassed by the method. Alternatively, the reference hydration value may be collected by a reference hydration measurement device 240, e.g. a body composition monitor (BCM) or the like.

Furthermore, method step M6 represents reading-in the at least one measured hydration value measured by the hydration measurement device 200 into the data set generating device 510, in particular by a reading device 210.

Method step M7 represents reading-in at least one reference hydration value determined from the reference hydration data source 250 into the data set generating device 510, in particular by the reading device 210.

As method step M8, generating a data set specific for the patient, using the data set generating device 510 is encompassed by the method according to the present invention. Generating the data set is based on the read-in measured hydration value, the at least one determined reference hydration value. The data set resulting therefrom is usable for determining or estimating a hydration value of the patient on or of at least one second body part or body region of the patient or for determining the overall hydration value of the patient when using the hydration measurement device 200 in the future.

In case, also device-specific was collected (see step M4a) the generation of the data set (step M8), using the data set generating device 510, is carried out specifically also for the hydration measurement device 200 by basing the generation also on the device-specific data.

Method step M9 represents storing the generated data set in a data set storage 220.

On the right-hand side of the Fig. 1 further method steps S1 to S5 are indicated. They represent additional method steps as follows:
Step S1 represents providing a retrieving device 211 for the hydration measurement device 200 or using such a device.

Via the retrieving device 211, the data set specific for the hydration measurement device 200, which was stored in the data set storage 220 in method step M9, is retrieved in step S2. This is indicated by a dashed arrow.

In method step S3, when assessing the patient's hydration state at home, in the hospital or the like, at least one hydration value of the patient measured by the hydration measurement device 200 is read into the retrieving device 211.

Determining a hydration value of the patient of at least one second body part of the patient or determining the overall hydration value of the patient is represented by method step S4. The determining is based on, or using, the data set specific for the hydration measurement device 200 and on the read-in hydration value of the patient measured by the hydration measurement device 200. In particular, the measured hydration value may be an input value to the data set. In fact, it may be its only input value.

The hydration value of the patient determined may then be output or stored in method step S5.

It may be output to the patient via the hydration measurement device 200 or via another outputting device. Also the output to other parties, e.g. a member of the medical staff, relatives or the like, may be encompassed by the present invention. The output of the determined hydration value of the patient may be instead of the measured hydration value or in addition to it.

**Fig. 2** shows schematically simplified a set 100 according to the present invention in an exemplary embodiment. Data flows are indicated by arrows.

In the example of Fig. 2, the set 100 comprises a hydration measurement device 200. The hydration measurement device 200 is suitable and/or configured for measuring at least one hydration parameter of a first body part or body region, here the patient's wrist, as it is embodied as a wearable, alternatively, integrated in a wearable, here exemplarily in the patient's smart watch.

With this hydration measurement device 200 at least one hydration parameter of the patient is measured. This results exemplarily in obtaining a measured hydration value gathered from or at the wrist of the patient as an example for a first body part or body region.

A computing device 500 is also comprised by the set 100. The computing device 500 comprises software for executing the computational steps of the method according to the present invention, in particular as described with regard to the software herein. These computational steps may in particular be executed by a data set generating device 510 which is also comprised by the set 100, here exemplarily as part of the computing device 500.

The data set generating device 510 comprises at least one algorithm, or data, for generating a data set.

Collecting patient-specific data of the patient, e.g. blood pressure, body temperature, pulse, weight, height, sex, age etc., may be carried out via various means, all known to the skilled person. Patient-specific data may, in the example of Fig. 2, exemplarily be collected by a blood treatment apparatus 300 and/or a further patient's device 310, like a smart phone or tablet, a scale, an input means such as a keyboard, touch screen, or the like. It may in some embodiments be retrieved from storage devices comprised by or connected to such devices.

Whenever "patient-specific data" is mentioned herein this may include any parameter, data or value that may be determined, measured, collected, captured etc. on or with the patient.

The computing device 500, or in particular the data set generating device 510, may further be configured to determine, select or retrieve at least one reference hydration value from a reference hydration data source 250 (likewise comprised by the set 100 of Fig. 2) based on the patient-specific data of the patient.

By taking into account at least one read-in hydration value of the patient measured by the hydration measurement device 200 and/or the at least one reference hydration value determined from the reference hydration data source 250, the data set generating device 510 is configured to generate a data set specific for the patient.

The set 100 further comprises a data set storage 220, in which this generated data set may be stored. Hence, it comprises, or is configured to comprise, at least one data set generated based on read-in measured hydration values, reference hydration values, patient-specific data and/or device-specific data.

The data set is particularly generated in order to be used in connection with the hydration measurement device 200. It is particularly usable for determining or assessing a hydration value of the patient on or of at least one second body part or body region of the patient or for determining the overall hydration value of the patient when the patient subsequently uses the hydration measurement device 200 on the first body part or body region, e.g., in his/her daily routine. This may be at any time point in the future, during interdialytic monitoring etc.

This is described below in more detail:
As shown in the example of Fig. 2, the data set storage 220 may be an external storage device. It may be particularly implemented in a network or a cloud. This may allow storing data sets for different hydration measurement devices 200 of different patients in one location. The corresponding data set may be accessible by the hydration measurement device 200 to which it has been previously assigned.

In other embodiments, the data set storage 200 may be provided as part of other devices, e.g. as part of the computing device 500 or as part of the hydration measurement device 200. It may respectively be provided in the housing of the hydration measurement device 200. Hence, in several embodiments, the data set storage 220 may be provided as part of the hydration measurement device 200.

In the example of Fig. 2 the hydration measurement device 200, preferably having the features disclosed herein and being embodied in the form of a patient's smart watch, allows continuous(ly) monitoring of at least one parameter associated to the hydration state of the monitored patient, whereas the actually measured hydration state (at the patient's wrist, for example), is not necessarily the one of interest. Rather, the present invention allows to assess the hydration state at interest (related to the entire body, for example) with minimal technical effort, and little expenditure of time and cost). The hydration measurement device 200 may in turn comprise the retrieving device 211. This is indicated in Fig. 2 by a dotted bubble.

By the retrieving device 211, the data set specific for the hydration measurement device 200 stored in the data set storage 220 is retrieved.

Moreover, at least one hydration value of the patient measured by the hydration measurement device 200 is read into the retrieving device 211.

Determining a hydration value of the patient related to at least one second body part of the patient or determining the overall hydration value of the patient is based on the data set specific for the hydration measurement device 200 and on the read-in hydration value of the patient measured by the hydration measurement device 200.

The determined hydration value of the patient may be output via the hydration measurement device 200, e.g. on a display thereof, or via another outputting device to the patient.

### Reference Numerals

- 100: set

- 200: hydration measurement device
- 210: reading device
- 211: retrieving device
- 220: data set storage

- 240: reference hydration measurement device
- 250: reference hydration data source

- 300: blood treatment apparatus
- 310: further patient's device; smart phone; tablet

- 500: computing device
- 510: data set generating device

- M1 to M9: method step
- M4a: method step

- S1 to S5: additional method step

## Claims

1. A method for generating data sets for a hydration measurement device (200) to be used when measuring a hydration state of a patient, encompassing the steps:
- providing a hydration measurement device (200) for measuring at least one hydration parameter of a first body part or body region of the patient, the hydration measurement device (200) optionally being a wearable or optionally comprised by a wearable;
- measuring the at least one hydration parameter of the patient using the hydration measurement device (200) for obtaining a measured hydration value of or on that first body part or body region of the patient or providing such a measured hydration value;
- providing a data set generating device (510) comprising at least one algorithm, or data, for generating a data set;
- collecting patient-specific data of the patient;
- determining at least one reference hydration value from a reference hydration data source (250) based on the patient-specific data of the patient;
- reading-in the at least one hydration value of the patient measured by the hydration measurement device (200) into the data set generating device (510) by a reading device (210);
- reading-in the at least one reference hydration value determined from the reference hydration data source (250) into the data set generating device (510) by the reading device (210);
- generating a data set specific for the patient, using the data set generating device (510), based on the read-in measured hydration value, the at least one determined reference hydration value, wherein the data set is usable for determining a hydration value of the patient on or of at least one second body part or body region of the patient or for determining the overall hydration value of the patient when using the hydration measurement device (200) in the future;
- storing the generated data set in a data set storage (220), the data set storage (220) optionally being comprised by the hydration measurement device (200).

2. The method according to claim 1, wherein the reference hydration data source (250) is a reference hydration measurement device (240), and wherein the reference hydration value is at least one reference hydration value measured by the reference hydration measurement device (240) on or of at least one second body part of the patient.

3. The method according to claim 1 or 2, wherein additionally device-specific data of the hydration measurement device (200) is collected and wherein the generation of the data set, using the data set generating device (510), is carried out specifically also for the hydration measurement device (200) by basing the generation also on the device-specific data.

4. The method according to any one of the preceding claims, encompassing the steps:
- providing a retrieving device (211) for the hydration measurement device (200);
- retrieving, via the retrieving device (211), the data set specific for the hydration measurement device (200) stored in the data set storage (220);
- reading-in at least one hydration value of the patient measured by the hydration measurement device (200) into the retrieving device (211);
- determining a hydration value of the patient of at least one second body part of the patient or determining the overall hydration value of the patient based on the data set specific for hydration measurement device (200) and on the read-in hydration value of the patient measured by the hydration measurement device (200);
- outputting the determined hydration value of the patient via the hydration measurement device (200) or via another outputting device to the patient.
and optionally encompassing the steps:
- measuring another hydration value on or of at least one second body part or body region of the patient or measuring the overall hydration value of the patient while obtaining a measured reference hydration value of the patient;
- evaluating the data set based on the deviation between the measured reference hydration value and the determined hydration value of the patient;
- generating, if applicable, an up-date of the data set by the data set generating device (510) based on the result of the evaluation; and
- storing the updated data set in the data set storage (220).

5. The method according to any one of the preceding claims, wherein the hydration measurement device (200) is configured to measure via optical methods, in particular via optical spectrometry.

6. The method according to any one of the preceding claims, wherein the data set storage (220) is in signal communication with the retrieving device (211) of the hydration measurement device (200).

7. The method according to any one of the preceding claims, wherein the data set generating device (510) is programmed to use data analyzing methods, in particular when generating updated data set for the patient.

8. The method according to any one of the preceding claims, wherein the at least one algorithm, or data, is programmed to calculate a data set or an updated data set based on a multitude of values comprised by the patient-specific data, optionally weighted.

9. A hydration measurement device (200) for measuring at least one hydration parameter of a first body part of the patient, the hydration measurement device (200), optionally being a wearable or comprised by a wearable, configured for attributing a reference hydration value based on a data set in a data set storage (220) to the measured hydration value measured with the hydration measurement device (200) using at least one data set generated by the method according to any one of claims 1 to 8.

10. A hydration measurement device (200), preferably according to claim 9, for measuring at least one hydration parameter of a first body part of the patient, comprising a reading device (210) programmed for reading-in the data set from a data set storage (220) comprised by the hydration measurement device (200).

11. A hydration measurement device (200), preferably according to claim 9, for measuring at least one hydration parameter of a first body part of the patient, comprising a reading device (210) programmed for reading-in the data set from a data set storage (220) not comprised by the hydration measurement device (200).

12. A hydration measurement device (200), preferably according to any one of the claims 9 to 11, configured for executing the method, in particular as a computer-implemented method, according to any one of claims 1 to 8 for generating the data set based on at least a read-in measured hydration value, at least one reference hydration value, patient-specific data and device-specific data.

13. The hydration measurement device (200) according to any one of the claims 9 to 12, wherein the hydration measurement device (200) is configured to measure via optical methods, in particular via optical spectrometry.

14. A set comprising
- a hydration measurement device (200), preferably according to any one of claims 9 to 13;
and also comprising one or more of the following items:
- a reference hydration measurement data source (250); and/or
- a computing device (500) comprising software for executing the computational steps of the method according to any one of claims 1 to 8; and/or
- a data set storage (220) comprising, or configured to comprise, at least one data set generated based on read-in measured hydration values, reference hydration values, patient-specific data and/or device-specific data.

15. A computer program configured for carrying out or initiating the computational steps of the method according to any one of the claims 1 to 8 or a computer program-product having a program code or such a computer program stored on a machine readable carrier.
